# EUROPEAN PATENT APPLICATION

(11) **EP 3 298 957 A1**
(43) Date of publication of application: **28.03.2018**
(21) Application number: 17190967.4
(22) Date of filing: 13.09.2017
(51) Int. Cl.: A61B 5/00, G01G 19/00

(54) **PREDICTION METHOD AND DIETARY REGIME**

(30) Priority: 21.09.2016 EP 16306212
(71) Applicant: Mars, Incorporated, McLean, VA 22101 (US)
(72) Inventor: FLANAGAN, John, 30470 Aimargues (FR); HOURS, Marie Anne, 30470 Aimargues (FR); NGUYEN, Patrick, Nantes, Cedex 3, 44307 (FR); THORIN, Chantal, Nantes, Cedex 3, 44307 (FR); LECLERC, Lucie, Nantes, Cedex 3, 44307 (FR)
(74) Representative: Kapadia, Roxna

(57) **Abstract**

The present invention relates to a prediction method and to a diet and/or dietary regime. The invention relates to a method of determining if a canine animal is predisposed to being overweight in adulthood. The invention also relates to a diet and/or a dietary regime for a canine animal which is predisposed to being overweight in adulthood. The invention also relates to a method of preventing animals from being overweight in adulthood.

## Description

The present invention relates to a prediction method and to a diet and/or dietary regime.

Obesity and (overweight) is now a global concern for dogs and its prevalence is increasing. Obesity is the most common medical disease in companion animals and is associated with comorbidities such as orthopaedic disease, arthrosis, cardiorespiratory diseases and diabetes mellitus, as well as causing metabolic derangements, altered renal function and other respiratory dysfunction. In addition to these adverse effects on health, quality of life is poorer in obese and overweight dogs and lifespans can be shortened.

Over-feeding and reduced metabolic activity are regularly provided as the main reasons behind the increasing prevalence of overweight and obesity. Nevertheless, excess energy intake and reduced metabolic activity do not fully explain why some dogs become obese and some do not. To date, risk prediction factors for development of obesity in dogs are not known.

Given the obesity-related issues and the operating system of the energy balance, it would be a better strategy to prevent fat mass development than managing weight loss programs.

Accordingly, the present invention relates, in a first aspect, to a method of determining that a canine animal is predisposed to being overweight in adulthood, the method comprising:
(i) determining the birth weight of the canine animal and
(ii) determining the weight of said canine animal at 13-15 days of age and
(iii) determining the weight gain between the dates of (i) and (ii),
wherein a weight gain of 125% or more between the dates of (i) and (ii) determine that the canine animal is predisposed to being overweight in adulthood.

The time period in (ii) may be 14 days of age.

Adulthood may be defined as a time of from 2 years of age onward. Adulthood is typically referred to as the time in a canine animal's life when they are fully grown. By 2 years, all dogs should be fully grown. Dogs are often described with reference to three main size groups:
- small dogs are less than 25lbs when fully grown
- medium/large dogs are 25-60lbs when fully grown
- giant dogs are usually over 60lbs when fully grown.

Small dogs are typically fully grown by 12 months. Medium/large dogs are typically fully grown by 18 months. Giant dogs are typically fully grown by 24 months.

Accordingly, for small dogs, adulthood can also be defined as from 2 years of age onward or from 1 year of age onward. For medium/large dogs adulthood can be defined as from 2 years of age onward or from 18 months of age onward. For giant dogs, adulthood can be defined as from 2 years of age onward.

Thus, while the method of the first aspect of the invention is a determination as to the predisposition of being overweight in adulthood, the interventions proposed according to the second aspect of the invention and as otherwise describe here, can be implemented before and/or after adulthood is reached (preferably beforehand if a predisposition to being overweight is determined).

The life stages of puppyhood and adolescence are often used with reference to dogs, in particular with reference to the foodstuffs provided to meet their nutritional and energy requirements. Puppyhood typically ends at 6-18 months, depending on the animal size (small, medium/large, giant) as defined above. Adolescence starts after puppyhood ends, i.e. 6-18 months, depending on the animal size.

The term overweight includes obese/obesity. Overweight and obese dogs carry excessive accumulation of adipose tissue which is translated to a body weight >15% and >30% above ideal body weight, respectively. Ideal body weight is defined as the weight of the dog with a body condition score (BCS) rating of 5 on a 9 point scale, as described in the journal article by Laflamme D, in Canine Practice, July/August 1997, vol 22, No. 4, pages 10-15, entitled "Development and Validation of a Body Condition Score System for Dogs", incorporated herein by reference.

The steps of determining the weight of the animal at steps (i) and (ii) is by any method, including weighing the animal on standard weighing scales (electronic or other). Measurement of body weight is normally performed within the first 3 hours of life and is measured on a standard weighing scales capable of reading to +/- 1g. All other weighing should be performed at the same phase of the day for each animal, for example after the feed or prior to the feed.

The canine animal is preferably a domestic dog. Domestic dog breeds which are included in the present invention include, Labrador Retriever, Golden Retriever, Beagle, Chihuahua, Jack Russell Terrier, Pug, Labrador, Yorkshire Terrier, Dachshund, Rottweiler, Border Collie, Cavalier King Charles Spaniel, Carlino, Boxer, Poodle, Shih Tzu, Shetland Sheepdog, Teckel, Cocker Spaniel, German Shepherd, Maltese, Schnauzer, Staffordshire Bull Terrier, West Highland White Terrier, Cocker Spaniel, English Bulldog, including any potential cross-breeds of breeds included or not in the aforementioned list.

According to the method of the invention, the animal which has been determined to be predisposed to be overweight in adulthood may have a diet and/or a dietary regime and/or behavioural intervention put in place to maintain or achieve a healthy bodyweight of the animal. Such a diet and/or dietary regime may be low in fat, low in energy, provide satiating effects to the animal, may reduce anxiety and stress, may reduce signs of begging, may enhance quality of life and/or may enhance activity levels of the animal. Such a behavioural intervention may include increasing the activity/exercise of the animal.

Such a diet and/or dietary regime is described below and is the second aspect of the invention.

A second aspect of the invention relates to a diet and/or a dietary regime for a canine animal which has been determined as predisposed to being overweight in adulthood according to a method according to the first aspect of the invention.

The diet and/or dietary regime of the second aspect may be any diet and/or dietary regime, including those which are already known, and those of the future. The diet and/or dietary regime is preferably started or used as soon as the determination of the first aspect is made. As soon as may be immediately (the next meal) or within 1, 2, 3 or 4 weeks, although any time period is contemplated. As soon as may include waiting for any growth spurt which the canine animal is experiencing. The diet may be a single or varied diet. The dietary regime may be a daily, weekly, or monthly regime with a single or more than a single diet. The diet and/or dietary regime may have a certain or restricted number of calories, in particular a diet and/or dietary regime which is low in calories without under nutrition (or, in other words, while maintaining nutritional requirements).

Preferably, the diet and/or dietary regime and/or behavioural intervention is put in place for a length of time such that the canine animal meets the ideal weight for the breed of dog at the age, gender and sexual maturity. Such a weight is known in the art according to a body condition score on a 9 point scale (as described in Laflamme D, earlier referenced). The diet and/or dietary regime may be for a period of up to 4, up to 8, up to 6 or up to 16 or up to 32 weeks, up to a year or for longer, including the lifetime of the animal.

The diet and/or a dietary regime according to the second aspect of the invention may be/have on a dry matter basis:
- an energy density of less than 4100 kcal/kg and/or
- less than 20% or less than 15% fat and/or
- less than 25% or less than 20% carbohydrate and/or
- more than 30% protein and/or
- more than 5% or more than 10% total dietary fibre

Optional other components include one or more prebiotic, probiotic and/or any other ingredient. The precise diet and/or dietary regime will likely be determined on additional factors such as the breed, age, sex, activity level, body condition and other characteristics of the canine animal.

The present invention encompasses, as the or as part of the diet and/or dietary regime, any foodstuff which a canine animal may consume as part of its diet.

The foodstuffs are preferably a food product in their own right. Each may be a dry, semi-moist or a moist (wet) product. Wet food includes food that is usually sold in a container, such as a tin, pouch or tray and has a moisture content of 70% to 90%. Dry food includes food having a similar composition but with 5% to 15% moisture, often presented as small biscuit - like kibbles. Semi-moist food includes food having a moisture content of from above 15% up to 70%. The amount of moisture in any product may influence the type of packaging that can be used or is required. The food product, of any moisture level may be ready-to-eat.

The foodstuff encompasses any product that a canine animal consumes in its diet. Thus, the foodstuff may include the standard food products as well as food products for companion dogs, such as food snacks (for example snack bars, cereal bars, snacks, treats, biscuits and sweet products). The foodstuff may be a cooked product. It may incorporate meat or animal-derived material (such as beef, chicken, turkey, lamb, fish, blood plasma, marrowbone, etc or one or more thereof). Alternatively the foodstuff may be meat-free (preferably including a meat substitute such as soya, maize gluten or a soya product in order to provide protein). The foodstuff may contain additional protein sources such as soya protein concentrate, milk, protein, gluten, etc. The foodstuff may also contain starch, such as one or more grains (e.g. wheat, corn, rice, oats, barley, etc) or may be starch-free. The foodstuff may incorporate or be a gelatinised starch matrix. The foodstuff may incorporate one or more types of fibre such as sugar beet pulp, chicory pulp, chicory, coconut endosperm fibre, wheat fibre etc. The foodstuff can also be newly designed products currently not available. The most suitable foodstuff may be a product as described herein which is sold as a pet food, in particular a pet food for a domestic dog. It may be convenient to provide the foodstuff in a dry format, such as dried ready-to-eat cereal products (often referred to as kibbles).

The diet may contain ingredients which have a satiating effect, in particular a weight maintenance/weight loss diet for puppies which have a satiating effect. A maintenance diet with satiating effect may be particularly favoured as such a diet would be acceptable to reduce satiety in puppies, without reducing calories and without affecting growth. Puppy weight loss/gain/management diets would preferably be tailored to a diet based around the size of the dog groups (small, medium/large, giant, as described before), or based on the dog breed or individualised per puppy.

The foodstuff is preferably nutritionally complete so that the practice of the invention may provide a suitable nutritionally complete diet and/or dietary regime for the animal.

Optionally, the foodstuff may be multi-component. The multi-component foodstuff may comprise a dried ready-to-eat cereal product. The multi-component foodstuff may only comprise such dried ready-to-eat cereal products. Alternatively, the multi-component foodstuff may comprise a dried ready-to-eat cereal product and a wet or semi-moist product. The multi-component foodstuff may comprise individual packages of food which, when all individual packages are fed over a period of time, such as 1 day, 2 days or one week, provide the diet and/or dietary regime according to the invention. The individual products may be packaged as discussed below.

The foodstuff is preferably packaged. In this way the consumer is able to identify, from the packaging, the ingredients and content of the product and confirm that it is suitable for the particular animal in question. The packaging may be metal (usually in the form of a tin or flexifoil), plastic (usually in the form of a pouch or bottle), paper or card. The amount of moisture in any product may influence the type of packaging, which can be used or is required. The foodstuff may be available as a "kit" or "pack" wherein different or the same food compositions are individually packaged and these packages are somehow joined together, for example in a box and/or with overarching packaging for the two or more packages of food compositions.

This invention solves the problem by the identification of elevated risk of overweight/obesity in dogs at a young age. Specifically, puppies which increased their body weight by more than 125% in the first 2 weeks of life were found to be obese at 2 years of age.

The invention can be used by dog breeders and dog owners to evaluate the risk of obesity of puppies from a very young age. In this way, the breeders and owners will be alerted to the elevated level of risk which pertains to these dogs and will be sensitive to any minor increases in their dog's body fat. By increasing the owner's awareness of their dog's overweight/obesity development risk, preventative measures (such as reduction in calorie allocation or increase in physical exercise) can be employed to eliminate the development of the effects of overweight/obesity.

The present invention also provides a device and system for the method according to the first aspect of the invention, of determining that a canine animal is predisposed to being overweight in adulthood. The device and/or system enable a user, such as a carer or owner to evaluate the weight gain and (preferably) take action by themselves or with the aid of a healthcare professional/vet to ensure a healthy bodyweight is achieved and maintained.

The method of the invention can be carried out with the aid of a mobile device, such as a tablet or smartphone.

The device of the invention is likely to be configured to accept a, user input into an application which is executed by the device comprising a processor, the user input comprising the birthweight of the animal according to step (i) of the first aspect of the invention. A further user input is configured to be accepted by the application on the device, the further user input being the weight of the animal according to step (ii) of the method of the invention.

The application on the device automatically (or on request) performs an analysis of step (iii), i.e. determining the weight gain between the input details in steps (i) and (ii) of the method of the invention. The analysis provides the weight gain of step (ii) and provides output information wherein the weight gain is 125% or more as set out according to the method of the first aspect of the invention.

The device provides a message with the output of step (iii) which may be a warning, depending on the result of step (iii).

In one embodiment, the results of the method are provided by the application in a user interface.

A device may be used. The device comprises a processor that executes an application that directs the device to provide data fields for entry of information related to step (i) and step (ii) and the application uses the processor to evaluate the risk of the animal being predisposed to overweight in adulthood.

The device may be a tablet, smartphone, desktop computer, laptop computer or personal digital assistant.

A system is also provided. The system comprises a database connected to a remotely located device comprising a processor executing an analysis that evaluates a determination according to the method of the first aspect of the invention.

An option of the invention is an easily navigable application, e.g. online, to determine a method according to the first aspect of the invention.
The device and/or application may provide a recommendation of nutritional suggestions according to the diet and/or dietary regime according to the second aspect of the invention.

All preferred features of the first aspect of the invention, such as the dog breeds, also apply to the second aspect.

A third aspect of the invention relates to a method of preventing an animal being overweight in adulthood comprising:
(i) determining the birth weight of the canine animal and
(ii) determining the weight of said canine animal at 13-15 days of age and
(iii) determining the weight gain between the dates of (i) and (ii),
   wherein a weight gain of 125% or more between the dates of (i) and (ii) determine that the canine animal is predisposed to being overweight in adulthood, and
(iv) administering a maintenance diet for a maintenance period and/or
(v) administering a weight adjustment diet for a weight adjustment period.

Steps (i), (ii) and (ii) of the method of the third aspect of the invention are the same as those according to the first aspect of the invention.

Where the animal is determined to be predisposed to being overweight in adulthood, the weight adjustment diet may be a weight loss diet. For other uses, the weight adjustment diet may be a weight gain diet.

Weight maintenance, weight loss and weight gain diets are exemplified in the following table.

| Diet | Energy Density kcal / kg | Fat % | Carbohydrate % | Protein % | Crude Fibre % | Calcium : Phosphorus |
|---|---|---|---|---|---|---|
| Puppy/Adolescence Weight Maintenance | 3800-4200 | 20 - 22 | 18 - 50 | 25 - 35 | 2-15 | 1:1.5 |
| Puppy/Adolescence weight loss | 3000-4000 | 11 - 20 | 25 - 50 | 27-38 | 4-18 | 1:1.5 |
| Puppy/Adolescence Weight Gain | 4000-4500 | 22 - 26 | 15-50 | 25-40 | 2-12 | 1:1.5 |
| Adult weight Maintenance | 3400-4000 | 12 - 20 | 40 - 60 | 15 - 28 | 2 - 15 | 1:1.2 |
| Adult weight loss | 2500-3500 | 7 - 12 | 45 - 60 | 25 - 35 | 8 - 18 | 1:1.2 |
| Adult Weight gain | 3800-4500 | 20 - 30 | 30 - 60 | 18 - 33 | 2 - 12 | 1:1.2 |

The terms 'adolescent' and 'junior' dog have the same meaning. The term 'adolescent' is used in this text.

An adult diet is a diet for an animal that has fully grown. As such, the animal can be described as requiring a weight maintenance diet. A weight gain diet is often appropriate when the animal is growing, for example in puppyhood and/or adolescence. An overweight puppy, adolescent or adult may need a weight loss diet.

The method of the third aspect of the invention may further comprise determining a maintenance body weight, comparing the maintenance body weight to a healthy body weight to calculate a body weight deviation, and administering a weight adjustment diet if the body weight deviation is 5% to greater. The maintenance body weight can be determined when the animal enters adulthood or any time afterwards. The maintenance body weight can be determined by any method, including weighing the animal on standard weighing scales (electronic or other).

The weight adjustment diet is a weight loss diet if the body weight deviation is above the healthy body weight.

The weight adjustment diet is a weight gain diet if the body weight deviation is below the healthy body weight.

The method of the third aspect of the invention may be a therapeutic method, preferably a therapeutic method to prevent dogs being overweight in adulthood and maintaining good health.

A fourth aspect of the invention relates to a diet and/or a dietary regime for use in preventing a canine animal being overweight in adulthood, wherein the diet and/or dietary regime is administered after a method is carried out according to the first aspect of the invention. The diet and/or dietary regime may be any as herein described. The diet and/or dietary regime may be for a therapeutic use, it may be a therapeutic use for preventing obesity and/or a therapeutic use for maintaining good health.

All preferred features of the first and/or second aspect of the invention, apply to the third and fourth aspects of the invention *mutatis mutandis.*

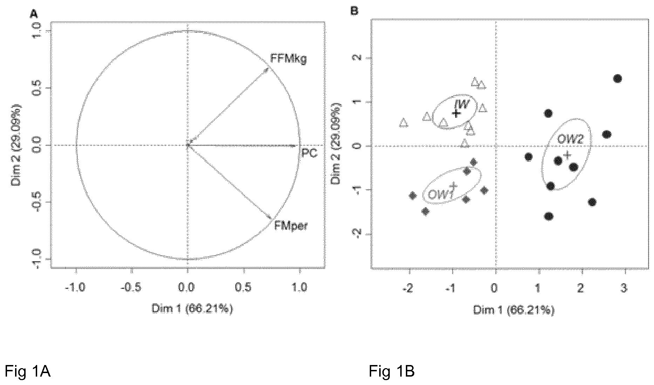

The invention is described with reference to the figures, in which
Figure 1 shows outputs of the PCA followed by the hierarchical clustering on principal components (HCPC), assessed on 24 female Beagle dogs aged 24 months. Groups were defined as ideal weight (IW, n=9), slightly overweight (OW1, n=6) or severely overweight (n=9). A: Factor map of principal and supplementary variables. The principal variables (solid lines) are fat-free mass (FFM, kg), fat mass proportion (FM%, %,) and pelvic circumference (PC, cm). B: Confidence ellipses (95% confidence level) around the groups identified by (HCPC).

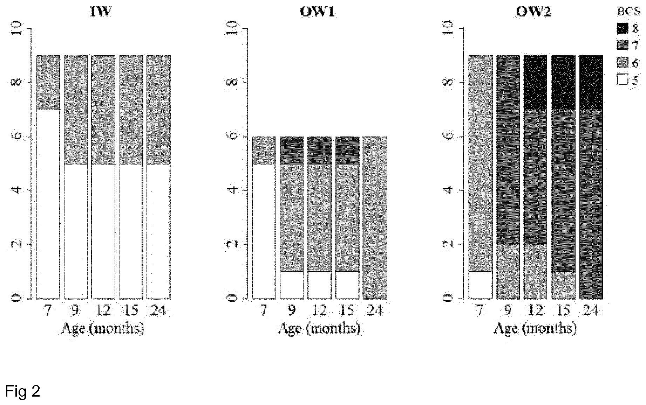

Figure 2 shows the number of dogs of each BCS at considered ages. Groups were defined as ideal weight (IW, n=9), slightly overweight (OW1, n=6) or severely overweight (OW2, n=9).

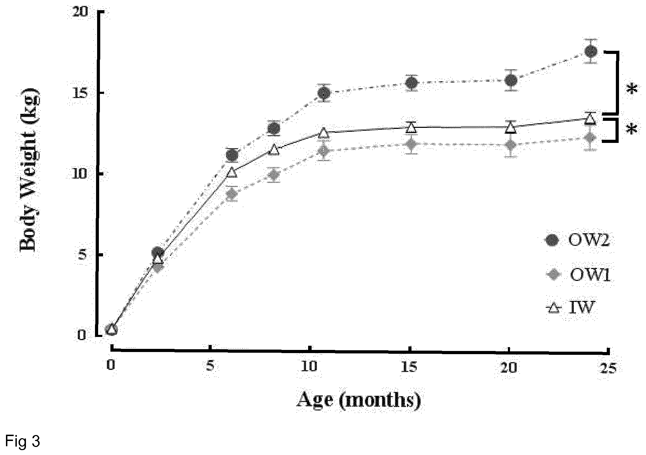

Figure 3 shows the body weight as a function of time in 24 female Beagle dogs. Values represent means of groups, while error bars represent SEM. Groups were defined as ideal weight (IW, n=9), slightly overweight (OW1, n=6) or severely overweight (OW2, n=9). * significant difference (p < 0.05) between groups as identified by a linear regression model.

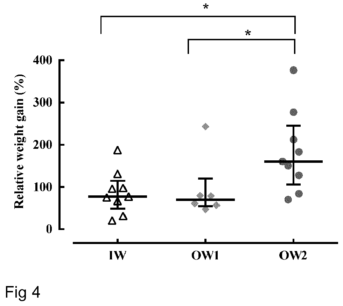

Figure 4 shows a scatter plot of GR_{2W} (%) of three groups, during the two first weeks of life. Groups were defined as ideal weight (IW, n=9), slightly overweight (OW1, n=6) or severely overweight (OW2, n=9). Data are presented by groups, the line represents the median of the group; error bars represent the interquartile range. * significant difference (p < 0.05) between groups as identified by a linear regression model.

The invention is described with reference to the following example:

### Example

**Summary:** In order to identify factors which may help explaining development of adult overweight status in dogs, a longitudinal study of 2 years was conducted on 24 female Beagle dogs of the same age, sexual status and raised under identical environmental conditions. To analyse the data, three groups of dogs were constituted: i) ideal weight (IW, n=9), ii) slightly overweight (OW1, n=6) and iii) severely overweight or obese (OW2, n=9), according to a PCA at 2yrs. of age, on the values of fat-free mass, %fat mass and pelvic circumference. The relevant predictive factors of a marked overweight status in adult dogs were statistically selected among the following factors: parental characteristics, growth pattern, energy balance and plasma factors.

**Results**: Logistic regression analysis highlighted that the % of weight gain during the 2 first weeks of life, the neonatal growth rate, was higher in the OW2 group than in the IW (p=0.015) and the OW1 groups (p=0.040). Seventy percent of dogs displaying a neonatal growth rate greater than 125%, belonged to the OW2 group.

**Conclusion:** The present study established that severely overweight adult dogs had a higher neonatal growth rate.

The canine animals were measured using standard weighing scales and the birth weight measured within 24 hours of birth.

### Background

Overweight and obese dogs carry excessive accumulation of adipose tissue which is translated to a body weight (BW) >15% and >30% above ideal BW, respectively. The main reason for excess weight gain in healthy dogs is a positive imbalance between energy intake and energy expenditure. Some obesity-related outcomes are reversible by restricted energy intake and by increased activity through a weight loss program. However, only half of the dogs entering such program reaches their target body weight and among them, only half of them succeed in maintaining their optimal body weight over the long term. Among the plasma factors related to energy intake, some of them were reported to differ between obese dogs and normal weight dogs, such as insulin, ghrelin, leptin and adiponectin. Obesity in dogs also results in low-grade systemic inflammation which may contribute to the genesis of metabolic disorders.

Numerous investigations have identified risk factors which were associated with excess weight, in overweight or obese but otherwise healthy dogs in various countries. The main reported risk factors are neutering especially in females, high feeding frequency, sedentary lifestyle and specific breeds or cross-breeds.

The aims of the study were (i) to show that spontaneous differences in weight gain could occur among dogs raised in the same conditions (of age, sex, sexual status, breed, diet and environment) and (ii) to identify early predictive factors such as parental and neonatal characteristics, energy intake or expenditure, or plasma biomarkers, associated with overweight status in adulthood.

### Methods

### Animals

Twenty-four female Beagle dogs were studied from birth to 24 months of age. They were born from 10 different mothers and 7 different fathers, housed in the same breeding centre (ISOQUIMEN, Barcelona, Spain), weaned at 10 weeks, and neutered at the age of 8 months.

All dogs received an annual veterinary check-up, were vaccinated and received worming treatments every 3 months

### Housing

Throughout the study, the dogs lived in the same environment and were fed in the same manner. Prior to weaning, puppies and mothers were housed by litter in the breeding centre of Isoquimen. After weaning, dogs were relocated to Oniris (Nantes, France) and were housed in pairs in closed outdoor enclosures of 4m². During feeding time, half the dogs were fed in their enclosures, while the remaining dogs were housed in two 31m² outdoor enclosures. The outdoor enclosures disposed of a sheltered place to sleep.

Dogs were taken for a walk at least 15 min twice a week and had access to 1 h/day of free time in a closed garden of 400m² enriched with agility equipment.

### Diet

All diets were the same diet (protein: 30%, fat: 22%, 4012 kcal/kg) and it was offered *ad libitum* to both mothers and puppies from conception to weaning.

### Data analysis

All statistical analyses were performed using the R software. Graphs were prepared using GraphPad Prism software. Significant differences have been considered for a risk of first type error of 0.05.

As a principal component analysis (PCA) has to be performed on quantitative variables, it was conducted on the three following variables: pelvic circumference (PC), fat free mass (FFM) and proportion of fat mass (FM%) at the age of 24 months (FactoMineR package, in R software). Consequently, BCS, which is a discrete variable, was added to the PCA as a supplementary variable as well as the parental and gestational variables.

A hierarchical classification on principal components was conducted on the previous variables to allot the dogs into three distinct groups.

Logistic regression analyses were performed on the 3 groups, by pairs (IW-OW1; 0W1-OW2, IW-OW2) to discriminate the groups by
i) parental and gestational variables,
ii) BCS, FM% or FFM before 2years old

An estimation of an accurate threshold was processed after the logistic regression analysis, on the identified discriminant factors. The goodness of fit was explored through an analysis of deviance table+.

### Results

All dogs remained healthy during the study and their serum biochemistry values remained within normal ranges.

### Constitution of 3 groups based on the dogs' biometric data at 24 months of age

In order to categorize dogs according to its overweight status, a PCA was performed on PC (cm), FM% (%) and FFM (kg) at 24 month of age. The two first axes explained 95.3% of the total variation. A hierarchical clustering on principal components revealed 3 well separated groups, which could be described as: dogs with ideal (optimal) weight (IW, n=9), slightly overweight dogs (OW1, n=6) and severely overweight or obese dogs (OW2, n=9).

At 24 months, the BW, FFM, FM% and PC values were significantly higher in the OW2 group than those in the OW1 group (p<0.001, p<0.001, p=0.009, p<0.001, respectively). The BW, FM% and PC were significantly higher in the OW2 group than in the IW groups (all p<0.001). FFM was significantly lower in the OW1 group than in the IW group (p=0.010), whereas FM% was significantly greater in the OW1 compared to the IW group (p<0.001). The distribution of BCS in the groups at 24 months was in line with the groups' characteristics. Among the groups, 6 dogs in the IW group had a BCS of 5/9, all dogs in the OW1 group displayed a BCS of 6/9 and all dogs of the OW2 group had a BCS superior to 6/9. The withers height was significantly higher in the OW2 (42 (1.5) cm) group than those in the IW (40.2 (1.4) cm; p=0.037) and the OW1 groups (39.0 (2.5) cm; p=0.003). The withers height was not significantly different between the IW and the OW1 groups (p=0.201).

Excess weight above optimal weight was estimated for each group as described in the methods. The percentage of excess weight of each group was found to be significantly higher in the OW2 group than in OW1 (p=0.002) and the IW groups (p<0.001), and higher in the OW1 group than in the IW group (p=0.001).

### Physiological periods of weight gain

### • Birth to weaning

The BW_{b} (IW: 0.44 (0.7) kg; OW1: 0.47 (0.14) kg; OW2: 0.39 (0.06) kg) did not differ among the groups.

The growth rate during the two first weeks of life (GR_{2W})(IW: 87.1 (50.5) %; OW1: 94.4 (73.8) %; OW2: 182.4 (30.2) %), was significantly higher in the OW2 than in the IW (p=0.015) and the OW1groups (p=0.040). A logistic regression analysis highlighted that GR_{2W} discriminated the OW2 from the IW (p=0.051) groups. Moreover, a GR_{2W} of 125% during the first 2 weeks of life was detected as an accurate threshold to predict OW2 status in adulthood. Hence, among the 10 dogs which displayed a GR_{2W} higher than 125%, 7 belonged to the OW2 groups in adulthood (2 to IW and 1 to OW1). Furthermore, GR_{2W} was significantly correlated with FM% (data not shown, p=0.038) independently of groups.

### Discussion

This is, to our knowledge, the first longitudinal study in growing dogs that aimed to identify predictive factors which could explain overweight status in adulthood.

### 1. First aim: Spontaneous excess weight gain in a part of an homogenous growing population and raising conditions

The first aim of this study was to determine whether spontaneous differences of weight gain could occur in a homogenous population of dogs fed *ad libitum.* Our population was composed of female Beagle dogs of the same age, sterilised and raised in identical conditions from conception to 2 years of age. The Beagle breed was chosen because its propensity to develop obesity, in addition to other risks such as age, sex, sexual status and environmental conditions.

### 1.1 Same conditions lead to excess weight at 2 years. of age

Dogs were allotted, a posteriori, into 3 groups according to FM%, FFM and PC at 24 months of age. The dogs that spontaneously became severely overweight or obese (BCS of 7 or 8) accounted for 37.5% of the entire population (OW2), whereas the dogs with an optimal weight (OW1, BCS of 5 or 6), also accounted for 37.5% of the population. The proportion of overweight dogs (62.5%) is in line with the reported prevalence of overweight dogs worldwide. Given that the dogs were fed *ad libitum,* it is worthy to note a subset of the population did not become overweight. These results confirm that some dogs could be more susceptible to gain body fat than others of the same breed and housed under the same environmental conditions.

### 2. Second aim: Identify explanation/prediction factors of the excess weight gain

The second aim of this study was to identify, as early as possible, predictive markers of overweight status in adulthood.

### 2.1 "Neonatal" conditions

In contrast to human and animal studies, body weight at birth was neither correlated to the overweight status, fat-free mass at adulthood nor correlated to parental body weight.

### 1. Predictive factor: growth rate on the 2 first weeks of life

Given that the normal weight for neonatal puppies at 10 days of age is twice that at birth, the growth rate in the first 2 weeks of life (GR_{2W}) was assessed. As 78% percent of dogs which had a GR_{2w} greater than 125% belonged to the OW2 group, GR_{2w} was identified as the earliest predictive factor of the adult overweight status in the present study.

### Conclusion

The neonatal finding can be used by breeders to identify the dogs which should be dietary restricted. The adolescence findings might be used by veterinarians to assess the risk of becoming overweight in adulthood and to deliver specific nutritional advice.

### List of abbreviations

- BCS: Body condition score
- BW: Body weight
- BW_{b}: Body weight at birth
- FM%: Percentage fat mass
- FFM: Fat free mass
- GR_{2W}: Growth rate in the 2 first weeks of life
- PC: Pelvic circumference

## Claims

1. A method of determining that a canine animal is predisposed to being overweight in adulthood, the method comprising:
(i) determining the birth weight of the canine animal and
(ii) determining the weight of said canine animal at 13-15 days of age and
(iii) determining the weight gain between the dates of (i) and (ii),
wherein a weight gain of 125% or more between the dates of (i) and (ii) determine that the canine animal is predisposed to being overweight in adulthood.

2. A method as claimed in claim 1, wherein the time period in (ii) is 14 days of age.

3. A method as claimed in claim 1 or claim 2, wherein adulthood is from 2 years of age onward.

4. A method as claimed in any one of claims 1 to 3, wherein overweight is obese.

5. A method according to claim 1, wherein the canine animal is a domestic dog.

6. A method according to any one of claims 1 to 5 wherein the animal is determined to be predisposed to be overweight in adulthood and a diet and/or a dietary regime put in place to maintain or achieve a healthy bodyweight.

7. A diet and/or a dietary regime for a canine animal which has been determined as predisposed to being overweight in adulthood according to a method as claimed in any one of claims 1 to 6.

8. A diet and/or a dietary regime as claimed in claim 7, wherein the dietary regime contains on a dry matter basis
• an energy density of less than 4100 kcal/kg and/or
• less than 20% or less than 15% fat and/or
• less than 25% or less than 20% carbohydrate and/or
• more than 30% protein and/or
more than 5% or more than 10% total dietary fibre.

9. A method of preventing an animal being overweight in adulthood comprising:
(i) determining the birth weight of the canine animal and
(ii) determining the weight of said canine animal at 13-15 days of age and
(iii) determining the weight gain between the dates of (i) and (ii),
wherein a weight gain of 125% or more between the dates of (i) and (ii) determine that the canine animal is predisposed to being overweight in adulthood, and
(iv) administering a maintenance diet for a maintenance period and/or
(v) administering a weight adjustment diet for a weight adjustment period.

10. The method of claim 9, wherein the weight adjustment diet is a weight loss diet or a weight gain diet.

11. The method of claim 9, further comprising determining a maintenance body weight, comparing the maintenance body weight to a healthy body weight to calculate a body weight deviation, and administering a weight adjustment diet if the body weight deviation is 5% or greater.

12. The method of claim 11, wherein the weight adjustment diet is a weight loss diet if the body weight deviation is above the healthy body weight.

13. The method of claim 11, wherein the weight adjustment diet is a weight gain diet if the body weight deviation is below the healthy body weight.

14. The method of claims 9 to 13 may be a therapeutic method, preferably a therapeutic method to prevent obesity.

15. A diet and/or a dietary regime, for use in preventing a canine animal being overweight in adulthood, wherein the diet is administered after a method of claims 1 to 6 is carried out.
